# EUROPEAN PATENT APPLICATION

(11) **EP 2 281 597 A1**
(43) Date of publication of application: **09.02.2011**
(21) Application number: 09167336.8
(22) Date of filing: 06.08.2009
(51) Int. Cl.: A61M 16/08

(54) **Respiratory nasal mask with a rotatable gas connector having one or several venting ports**

(71) Applicant: Air Liquide Medical Systems, 92182 Antony Cedex (FR)
(72) Inventor: Rivetti, Alberto, 25038, ROVATO (BS) (IT); Alberici, Luca, 25128, BRESCIA (IT); Sandoni, Giuseppe, 25124, BRESCIA (IT)
(74) Representative: Pittis, Olivier

(57) **Abstract**

A gas connector for respiratory mask, especially for nasal mask, comprising a curved hollow main body (8) comprising an internal passage (8c) with an inlet orifice (8a) and an outlet orifice (8b), said internal passage (8c) comprising at least a first linear portion (16) of first axis (A-A) comprising the inlet orifice (8a), a second linear portion (18) of second axis (B-B) comprising the outlet orifice (8b), and an intermediary portion (17) situated between said first (16) and a second (18) linear portion and comprising at least one venting port (3) traversing the wall of said intermediary portion (17) and having a third axis (b-b), **characterized in that** the third axis (b-b) of each venting port (3) is parallel to the second axis (B-B) of the second linear portion (18) of said internal passage (8c). The mask of the invention can be used in the treatment of respiratory conditions or diseases, such as obstructive sleep apnea or similar.

## Description

The invention concerns a respiratory mask, in particular a nasal mask, with an improved connector for supplying pressurized gas to the mask and venting expired CO₂-containing gases, for use in the treatment of respiratory conditions or diseases, such as obstructive sleep apnea.

Nasal masks are commonly used for delivering non-invasive positive pressure ventilation (NPPV) or for nasal continuous positive airway pressure (N-CPAP) therapy of sleep disordered breathing (SDB) conditions, such as obstructive sleep apnea (OSA).

Nasal masks deliver a flow of breathable gas for or to assist patient respiration, especially during the night, i.e., when the patient is sleeping.

Such a mask assembly typically comprises a rigid or semi-rigid hollow shell defining a breathing chamber that receives at least a part of the patient's nose and further comprising a soft face-contacting cushion that comes into contact with the patient face, and a fore-head support and a headgear for correctly positioning, maintaining and/or securing the mask on the head of a patient. An example of a nasal mask of this kind is given by EP-A-462701.

The mask assembly is secured to the wearer's head by straps or similar forming a headgear that can be adjusted to pull the mask against the face with sufficient force to achieve a gas tight seal between the mask and the wearer's face. The headgear is usually connected to the mask by means of connection means, such as slots arranged in the shell walls as taught by EP-A-462701, EP-A-874667 or WO-A-00/57942, or by means of more complicated release mechanism as disclosed by EP-A-1972357. Indeed, it is important for the patient's comfort and for getting an efficient treatment that gas leaks between the face cushion and the patient's face are avoided or at least very limited, because if any leak appears, then it can result in the gas pressure supplied to the entrance of the patient's airway being below the therapeutic value and the treatment becoming ineffective or insufficient.

Typically, the gas under pressure is fed to the internal volume of the shell by a gas supply line, i.e., a flexible hose or similar, which convoys to the mask a respiratory gas under pressure, such as air, coming from a source of gas under pressure, typically a respiratory ventilator or similar.

The supply line is commonly connected to the mask by means of a gas connector having generally a hollow tubular structure for convoying the gas. Some connectors are connectable/disconnectable to the mask shell and/or rotatable with respect to the mask, whereas some others are made integral with the mask shell. These connectors can further comprise one or several exhalation ports or outlets for venting the gas expired by the patient. For instance, such gas connectors are disclosed by EP-A-1234593, US-A-4,274,406 and EP-A-462701.

The main role of a gas connector is to link the gas line to the mask and thus to ensure a continuous gas delivering to the patient. However, as already mentioned, another important role of a connector of this type is to be able to vent to the atmosphere, via outlet ports, the gas expired by the patient that contain a high concentration of CO₂.

Despite the fact that various connector configurations have already been proposed, the CO₂-containing gas venting is often poor, i.e., not very efficient, which leads to a re-breathing by the patient of expired CO₂ that has not been properly vented to the atmosphere. One can easily understand that such an expired-CO₂ re-breathing in not a good thing for the patient.

Further, during each expiration phases, when expired gases are vented by the venting ports of the masks of the prior art, they often create a noise that is very disturbing and problematic for the patient, especially during its sleep, as this noise that appears at each expiration phases can wake him up as well as anyone sleeping in the same room.

The main problem to be solved is to provide an improved gas connector for connecting a gas line to a respiratory mask, especially a nasal mask, used for treatment of sleep disorders, such as OSA or similar, with which the CO₂-containing gas venting is at least as efficient as and preferably more efficient than with the prior art connectors, and which generates less noise during the expiration phases of the patient than masks of the prior art.

The solution of the present invention is a gas connector comprising a curved hollow main body comprising an internal passage with an inlet orifice and an outlet orifice, said internal passage comprising at least a first linear portion of first axis A-A comprising the inlet orifice, a second linear portion of second axis B-B comprising the outlet orifice, and an intermediary portion situated between said first and a second linear portion and comprising at least one venting port traversing the wall of said intermediary portion and having a third axis b-b, **characterized in that** the third axis b-b of said at least one venting port is parallel to the second axis B-B of the second linear portion of said internal passage.

The connector according to the present invention can further comprise one or more of the following additional features:
- the first axis A-A of the first linear portion and the second axis B-B of the second linear portion are forming an angle α with respect to each other of between 60 and 110°, preferably an angle α of about 90°.
- each venting port has an inlet having a first size D1 and an outlet having a second size D2 such that: D1 > D2, preferably said first size D1 and second size D2 are diameters.
- the venting ports have a conical or tronconical shape.
- the intermediary portion comprises from 1 to 50 venting ports.
- the hollow main body comprises a first abutment arranged on the external wall of the first linear portion and a second abutment arranged on the external wall of the second linear portion.
- it is made of a polymeric material, preferably of plastic material.

Further, the present invention also deals with a respiratory mask comprising a shell comprising an internal chamber with a gas inlet port, **characterized in that** a connector according to the present invention is connected to said gas inlet port.

The shell further comprises a peripheral border and a cushion fixed to said peripheral border, said cushion having a central aperture for receiving at least part of the patient's nose, preferably said cushion comprises one or several flexible membranes.

Furthermore, the connector is mobile in rotation around the second axis B-B of the second linear portion with respect to the shell.

A preferred embodiment of a connector for nasal mask according to the present invention is shown in the enclosed Figures, among which :
- Figure 1 represents a front view of a nasal mask equipped with a gas connector according to the present invention,
- Figure 2 is enlarged side view of the connector of the mask of Figure 1, and
- Figure 3 shows the mask of Figure 1 equipped with a rotating connector according to the present invention in different angular positions.

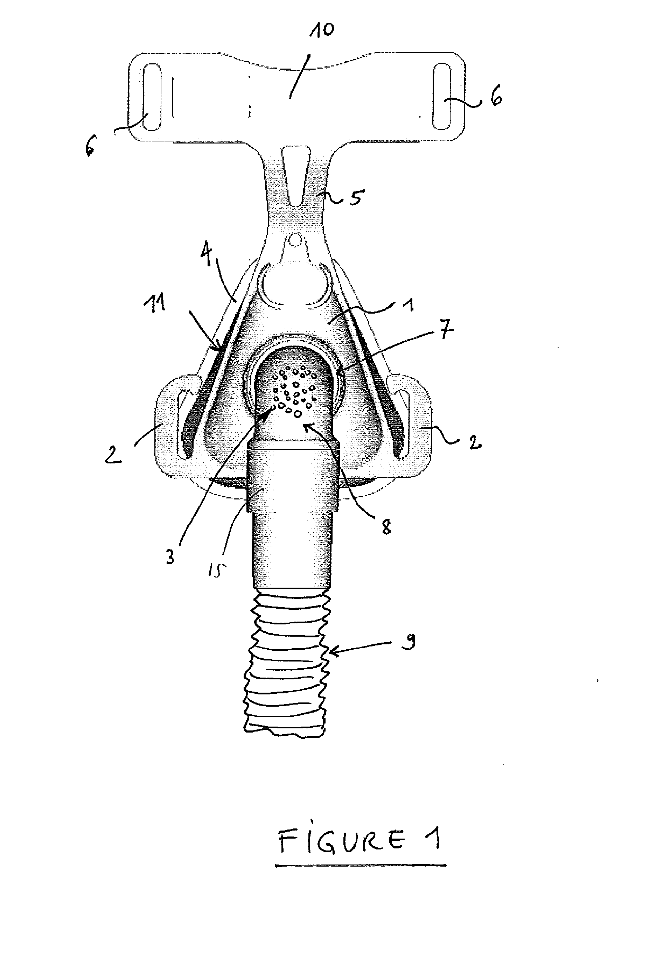

As illustrated in Figure 1, the respiratory mask according to the present invention comprises a rigid or semi-rigid hollow shell 1 defining an internal breathing chamber, wherein respiratory gas, such as air under pressure, is introduced via the inlet port 7 to which is connected a gas feeding line by means of a tubular gas connector 8 according to the present invention, which will be detailed below.

The inlet port 7 is arranged at the center of the shell 1 and through its wall thereby allowing air under pressure to be introduced in the breathing chamber.

In a preferred embodiment, the shell 1 has a general triangular shape as illustrated on Figure 1 and/or is preferably made of a polymer material, such as polycarbonate, polypropylene, ABS, nylon, polystyrene or similar.

More generally speaking, the shell 1 is configured so as to be able to receive at least a part of the patient's nose, when the mask is positioned on the patient's face. Actually, the patient introduces its nose first through the central aperture of the cushion 4 carried by the shell 1 and then into the internal volume of the breathing chamber of the shell 1, and breathes the pressurized gas contained therein.

The mask further comprises a fore-head support 10 connected to the shell 1 by means of a strip portion 5. A headgear with straps (not shown) is used for maintaining the mask in position on the head of the patient during the use of the mask, e.g., during a night when the mask is used for treating a sleep disorder, such as OSA. Such a mask structure is well known in the art and disclosed in many documents such as for instance EP-A-1334742, EP-A-462701, EP-A-1985327 or EP-A-956069. Several connection means 2, 6 integral with the shell 1 are provided for fixing the headgear to the shell 1. For instance, as shown in Figure 1, the frontal support 10 comprises two slots 6, whereas the shell comprises two hook-type parts 2 for fixing the straps of the headgear. Advantageously, hook-type parts 2 and the shell 1 are made of a unique molded structure, preferably a structure made of polymer that is thermo-molded for example. Of course, the hook-type parts 2 and slots 6 can have other forms or other types of connections means can be also used.

In order to ensure a tight positioning of the nasal mask on the patient's face and to increase the comfort for the patient, the peripheral border or edge 11 of the shell 1 comprises a cushion 4 made of soft, resilient, elastomeric material that comes into contact with the patient face, said cushion 4 having a central aperture for receiving at least part of the patient's nose. More precisely, the border 11 and the cushion 4 have a general triangular or saddle-shape structure so as to match the contours of the nasal region of the patients and hence the cushion 4 comprises, roughly speaking, an upper nasal bridge region, a lower region and cheek regions connecting the nasal bridge and lower regions. In order to improve the seal and tightness, the cushion 4 can comprise one or several membranes, preferably two membranes. Such cushion 4 and shell 1 structures are well-known in the art and taught by many documents, such as EP-A-1334742, EP-A-264772, EP-A-956069, EP-A-874667, US-A-2,931,356 or EP-A-1479406.

The shell 1 is fluidly linked to a gas supply line 9 fed with air under pressure by a respiratory device (not shown), by means of a tubular hollow gas connector 8, which delivers the respiratory gas, such as air under pressure into the breathing chamber of the shell 1.

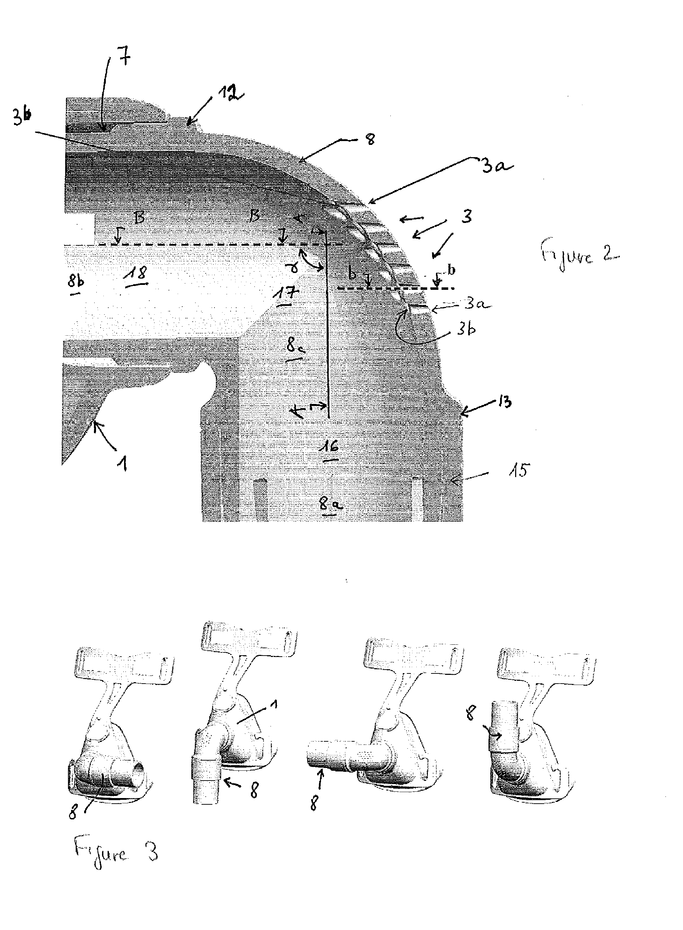

As detailed in Figure 2, the hollow gas connector of the present invention has a curved hollow main body 8 comprising an internal passage 8c, preferably having a circular section, having an inlet orifice 8a for receiving the gas delivered by the gas line 9 and an outlet orifice 8b for delivering the gas under pressure to the shell 1.

The internal passage 8c convoying gas under pressure from the inlet orifice 8a to the outlet orifice 8b, is formed by a first linear portion 16 of first axis A-A carrying the inlet orifice 8a; of a second linear portion 18 having a second axis B-B and carrying the outlet orifice 8b; and an intermediary curved portion 17 situated between the linear portions 16, 18 and comprising venting ports 3 having each a third axis b-b. The venting ports 3 are arranged through the wall of the intermediary curved portion 17 of the connector, i.e. they are traversing the wall of said intermediary portion 17 so as to establish a fluid communication between the inside of the connector and the ambient atmosphere situated outside the connector.

The internal passage 8c convoys the gas through said three portions 16, 17, 18.

According to the present invention, it is essential that the third axis b-b of the venting ports 3 is parallel to the second axis B-B of the second linear portion 18 so that at least part of the expired CO₂-containing gases, i.e. the expiration gaseous flow expired by the patient during the expiration phases, are efficiently vented to the atmosphere through the venting ports 3, whatever the angular position of the connector 8 with respect to the shell 1 as illustrated in Figure 3, and thus limiting the expiration noise.

Indeed, according to the invention, the connector is mobile in rotation around the B-B axis shown in Figure 2, i.e., with an angular motion of 360°. For instance, four different angular positions are represented in Figure 3. When orientating the axis b-b of the venting ports 3 parallel to the axis B-B, the flow of exhaled gases that follows the axis B-B of the second linear portion 18 is more efficiently vented through said ports 3.

Further, the axis A-A of the first linear portion 16 and the axis B-B of the second linear portion 18 are forming an angle (α) of between 70 and 110° with respect to each other, preferably of about 90°.

Advantageously, for further improving the efficiency of the gas venting and further limiting the expiration noise caused by the gas passing through the venting ports 3, it is advocated that the venting ports have a particular shape, i.e., a conical or tronconical form, as shown in Figure 2. In other words, each venting port 3 should have an inner diameter or size 3b that is greater than its outer diameter or size 3a. For instance, the number of venting port 3 can be of between 1 and 70, preferably between 1 to 50. Each venting port 3 can have an inner diameter 3b of between 0.5 and 4 mm and an outer diameter or size 3a of between 0.3 and 2 mm.

Furthermore, the hollow main body 8 comprises a first abutment 13 arranged on the external wall of the first linear portion 16 and a second abutment 12 arranged on the external wall of the second linear portion 18, for instance peripheral lips 12, 13 or similar, that are used for limiting the course of the connector when it is inserted in the central orifice 7 of the shell 1, on the one hand, and in connection part 15 that is linked to the flexible hose 9, on the other hand.

Of course, the connector comprises at its both ends carrying the inlet and outlet 8a, 8b, some connection means or structures (not detailed) allowing it to be connected to the hose 9 and to the shell 1.

Preferably, the connector is made of a polymeric material, more preferably of plastic material.

The nasal mask of the present invention can be used in a method for treatment of a respiratory disorder or condition, for instance in non-invasive positive pressure ventilation (NPPV) or in a nasal continuous positive airway pressure (N-CPAP) therapy of sleep disordered breathing (SDB) conditions, such as obstructive sleep apnea (OSA).

## Claims

1. Gas connector comprising a curved hollow main body (8) comprising an internal passage (8c) with an inlet orifice (8a) and an outlet orifice (8b), said internal passage (8c) comprising at least a first linear portion (16) of first axis (A-A) comprising the inlet orifice (8a), a second linear portion (18) of second axis (B-B) comprising the outlet orifice (8b), and an intermediary portion (17) situated between said first (16) and a second (18) linear portion and comprising at least one venting port (3) traversing the wall of said intermediary portion (17) and having a third axis (b-b), **characterized in that** the third axis (b-b) of each venting port (3) is parallel to the second axis (B-B) of the second linear portion (18) of said internal passage (8c).

2. Connector according to claim 1, **characterized in that** the first axis (A-A) of the first linear portion (16) and the second axis (B-B) of the second linear portion (18) are forming an angle (α) with respect to each other of between 60 and 110°, preferably an angle (α) of about 90°.

3. Connector according to any one of the previous claims, **characterized in that** each venting port (3) has an inlet (3b) having a first size (D1) and an outlet (3a) having a second size (D2) such that : D1 > D2, preferably said first size (D1) and second size (D2) are diameters.

4. Connector according to any one of the previous claims, **characterized in that** the venting ports (3) have a conical or tronconical shape.

5. Connector according to any one of the previous claims, **characterized in that** the intermediary portion (17) comprises from 1 to 50 venting port (3).

6. Connector according to any one of the previous claims, **characterized in that** the hollow main body (8) comprises a first abutment (13) arranged on the external wall of the first linear portion (16) and a second abutment (12) arranged on the external wall of the second linear portion (18).

7. Connector according to any one of the previous claims, **characterized in that** it is made of a polymeric material, preferably of plastic material.

8. Respiratory mask comprising a shell (1) comprising an internal chamber with a gas inlet port (7), **characterized in that** a connector according to any one of the previous claims is connected to said gas inlet port (7).

9. Respiratory mask according to claim 8, **characterized in that** the shell (1) further comprises a peripheral border (11) and a cushion (4) fixed to said peripheral border (11), said cushion (4) having a central aperture for receiving at least part of the patient's nose.

10. Respiratory mask according to any one of claims 8 or 9, **characterized in that** the cushion (4) comprises one or several flexible membranes.

11. Respiratory mask according to any one of claims 8 to 10, **characterized in that** the connector is mobile in rotation around the second axis (B-B) of the second linear portion (18) with respect to the shell (1).

## Amended claims

### Amended claims in accordance with Rule 137(2) EPC.

**1.** Gas connector comprising a curved hollow main body (8) comprising an internal passage (8c) with an inlet orifice (8a) and an outlet orifice (8b), said internal passage (8c) comprising at least a first linear portion (16) of first axis (A-A) comprising the inlet orifice (8a), a second linear portion (18) of second axis (B-B) comprising the outlet orifice (8b), and an intermediary portion (17) situated between said first (16) and a second (18) linear portion and comprising at least one venting port (3) traversing the wall of said intermediary portion (17) and having a third axis (b-b), the third axis (b-b) of each venting port (3) being parallel to the second axis (B-B) of the second linear portion (18) of said internal passage (8c), **characterized in that** each venting port (3) has an inlet (3b) having a first size (D1) and an outlet (3a) having a second size (D2) such that: D1 > D2.

**2.** Connector according to claim 1, **characterized in that** the first axis (A-A) of the first linear portion (16) and the second axis (B-B) of the second linear portion (18) are forming an angle (α) with respect to each other of between 60 and 110°, preferably an angle (α) of about 90°.

**3.** Connector according to any one of the previous claims, **characterized in that** said first size (D1) and second size (D2) are diameters.

**4.** Connector according to any one of the previous claims, **characterized in that** the venting ports (3) have a conical or tronconical shape.

**5.** Connector according to any one of the previous claims, **characterized in that** the intermediary portion (17) comprises from 1 to 50 venting port (3).

**6.** Connector according to any one of the previous claims, **characterized in that** the hollow main body (8) comprises a first abutment (13) arranged on the external wall of the first linear portion (16) and a second abutment (12) arranged on the external wall of the second linear portion (18).

**7.** Connector according to any one of the previous claims, **characterized in that** it is made of a polymeric material, preferably of plastic material.

**8.** Respiratory mask comprising a shell (1) comprising an internal chamber with a gas inlet port (7), **characterized in that** a connector according to any one of the previous claims is connected to said gas inlet port (7).

**9.** Respiratory mask according to claim 8, **characterized in that** the shell (1) further comprises a peripheral border (11) and a cushion (4) fixed to said peripheral border (11), said cushion (4) having a central aperture for receiving at least part of the patient's nose.

**10.** Respiratory mask according to any one of claims 8 or 9, **characterized in that** the cushion (4) comprises one or several flexible membranes.

**11.** Respiratory mask according to any one of claims 8 to 10, **characterized in that** the connector is mobile in rotation around the second axis (B-B) of the second linear portion (18) with respect to the shell (1).
